(19) Europäisches Patentamt
European Patent Office
Office européen des brevets

(11) EP 0 770 613 B1

(12) EUROPÄISCHE PATENTSCHRIFT

(45) Veröffentlichungstag und Bekanntmachung des Hinweises auf die Patenterteilung:
**09.06.1999 Patentblatt 1999/23**

(51) Int Cl.$^6$: **C07D 417/04**, C07D 413/04, C07D 403/04, C07D 401/04, A61K 31/41, A61K 31/44, A61K 31/495

(21) Anmeldenummer: **96116069.4**

(22) Anmeldetag: **08.10.1996**

# (54) Substituierte Imidazolidin-2,4-dion-Verbindungen als Immunodulatoren

Substituted Imidazolidin-2,4-dione derivatives as immunomodulators

Dérivés substitués de l'imidazolidin-2,4 dione comme immunomodulateurs

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **27.10.1995 DE 19540027**

(43) Veröffentlichungstag der Anmeldung:
**02.05.1997 Patentblatt 1997/18**

(73) Patentinhaber: **Grünenthal GmbH**
**52078 Aachen (DE)**

(72) Erfinder:
- **Zimmer, Oswald, Dr.**
**52146 Würselen (DE)**
- **Böhlke, Horst, Dr.**
**52222 Stolberg (DE)**
- **Wnendt, Stephan, Dr.**
**52076 Aachen (DE)**
- **Geist-Rudolf, Cornelia, Dr.**
**52159 Roetgen (DE)**
- **Zwingenberger, Kai, Dr.**
**52076 Aachen (DE)**

(56) Entgegenhaltungen:

| | |
|---|---|
| **EP-A- 0 363 061** | **WO-A-92/07567** |
| **WO-A-95/02591** | **DE-A- 363 061** |
| **US-A- 4 746 669** | |

- **PHARMAZIE 1983, Bd. 38, Seiten 341-342, XP002022929 SATSANGI, R. K. ET AL.:**
- **J. MED. CHEM. 1975, Bd. 18, Seiten 846-849, XP002022930 PENG, G. W. ET AL.:**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 770 613 B1

**Beschreibung**

Die Erfindung betrifft substituierte Imidazolidin-2,4-dion-Verbindungen, Verfahren zu deren Herstellung sowie die Verwendung dieser Verbindungen in Arzneimitteln.

In der Pathogenese einer Vielzahl von schwerwiegenden Erkrankungen spielt die überschießende Bildung des Zy-tokins Tumor-Nekrose-Faktor-α (TNF-α) eine zentrale Rolle. Zu diesen Erkrankungen gehören Multiple Sklerose, Graft-versus-Host-Syndrom, Transplantatabstoßung, aphthöse Stomatitis, Erythema nodosum leprosum, Morbus Boeck, rheumatoide Arthritis und eine Reihe weiterer Erkrankungen, die mit entzündlichen Erscheinungen einhergehen. Ein bekannter Therapieansatz für diese Erkrankungen besteht in der generellen Unterdrückung der TNF-α Freisetzung durch Immunmodulatoren mit suppressivem Charakter, beispielsweise Dexamethason.

Bei Erkrankungen mit leukozytär dominierten Vaskulitiden postkapillärer Venolen, beispielsweise aphthöser Stomatitis, kutanem Lupus erythematodes, Pyoderma gangränosum und orogenitalen Ulcera bei Morbus Behçet, ist allerdings eine fokussierte Intervention vorzuziehen, um die Nachteile der generellen Immunsuppression zu vermeiden.

Als pathogenetische Faktoren gelten bei diesen Krankheiten endogene Mediatoren mit Wirkungen auf das Endothel und zirkulierende Leukozyten. Eine lokale Freisetzung von TNF-α und anderer Zytokine führt zu einer fokalen Erhöhung der Adhäsivität des Endothels gegenüber den Leukozyten, was maßgeblich zur Vaskulitidenbildung beiträgt [M. Clauss et al. in: Tumor Necrosis Factors, Herausgeber: B. Beutler, Raven New York 1992, S. 49-64]. Substanzen, die durch eine fokale Intervention die Veränderung des Endothels unterdrücken können ohne zugleich die spezifische zelluläre Immunabwehr zu blockieren, sind generellen Immunsuppressoren, wie Dexamethason, überlegen und können neue therapeutische Möglichkeiten eröffnen.

Die Klasse der Hydantoin-Verbindungen, zu der auch die erfindungsgemäßen Verbindungen gehören, wurde in der Vergangenheit intensiv erforscht. Es wurde eine Vielzahl von Derivaten synthetisiert, die beispielsweise in kosmetischen Artikeln Anwendung finden, als Insektizide oder Herbizide eingesetzt werden oder die Basis für Epoxidharze bilden.

Im pharmazeutischen Bereich sind für Hydantoin-Verbindungen insbesondere antikonvulsive, antiinflammatorische [J. Med. Chem. 8, 239, (1965); Arzneim. Forsch./ Drug Res. 27(II), 1942 (1977); Pharmazie 38, 341, (1983); J. Med. Chem. 28, 601, (1985)] und Antitumorwirkungen [J. Med. Chem. 18, 846, (1975), Arzneim. Forsch./Drug Res. 34(I), 663, (1984)] bekannt.

Die der Erfindung zugrundeliegende Aufgabe bestand in der Entwicklung von neuen, stabilen Immunmodulatoren, die nicht zu einer generellen Immunsuppression führen. Ferner sollten die zu entwickelnden Substanzen eine antivaskulitische Wirkung besitzen.

Es wurde nun gefunden, daß die an die zu entwickelnden Substanzen gestellten Anforderungen von bestimmten substituierten Imidazolidin-2,4-dion-Verbindungen erfüllt werden. Diese zur Klasse der Hydantoine gehörenden Verbindungen zeichnen sich durch eine starke immunmodulatorische Wirkung aus. Sie unterdrücken die Freisetzung von TNF-α, ohne zugleich zu einer generellen Blockierung der zellulären Immunabwehr zu führen. Die erfindungsgemäßen Verbindungen zeigen außerdem eine antivaskulitische Wirkung, die nicht ausschließlich auf die Hemmung der TNF-α Freisetzung zurückzuführen ist.

Gegenstand der Erfindung sind dementsprechend substituierte Imidazolidin-2,4-dion-Verbindungen der Formel I

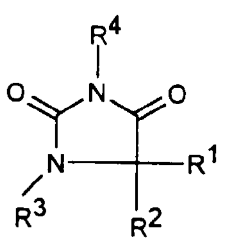

in der

$R^1$ $C_{1-6}$-Alkyl oder $C_{3-6}$-Cycloalkyl bedeutet,

$R^2$ $C_{1-6}$-Alkyl, Phenyl, $-(CH_2)_{1-3}$-Phenyl oder $-(CH_2)_{1-4}-COOR^5$ bedeutet oder

$R^1$ und $R^2$ zusammen $-(CH_2)_{4-6}-$, $-(CH_2)_2-O-(CH_2)_2-$ oder

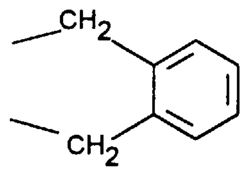

bedeuten,

$R^3$ H, $C_{1-5}$-Alkyl oder $-(CH_2)_{1-4}$-$COOR^5$ bedeutet,

$R^4$ ein Heteroaromat aus der Gruppe mit den Formeln

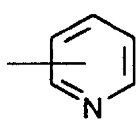 , 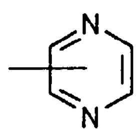 , 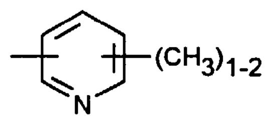 , 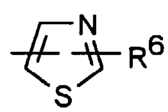 ,

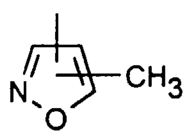 , 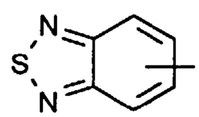 , 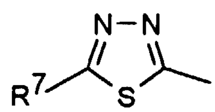

ist,

$R^5$ $C_{1-3}$-Alkyl darstellt,

$R^6$ H, $C_{1-4}$-Alkyl, Phenyl oder Benzyl bedeutet und

$R^7$ H, $C_{1-4}$-Alkyl oder Trifluormethyl bedeutet.

Bevorzugte substituierte Imidazolidin-2,4-dion-Verbindungen entsprechen der Formel I, in der

$R^1$ $C_{1-4}$-Alkyl oder $C_{3-4}$-Cycloalkyl bedeutet,

$R^2$ $C_{3-6}$-Alkyl, Phenyl, $-(CH_2)_{1-2}$-Phenyl oder $-(CH_2)_{1-2}$-$COOR^5$ bedeutet, oder

$R^1$ und $R^2$ zusammen $-(CH_2)_5$- oder

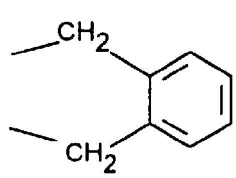

bedeuten,

$R^3$ H, $C_{1-4}$-Alkyl oder $-(CH_2)_{1-2}$-$COOR^5$ bedeutet,

$R^4$ ein Heteroaromat aus der Gruppe mit den Formeln

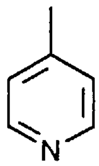, 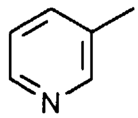, 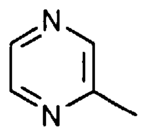, 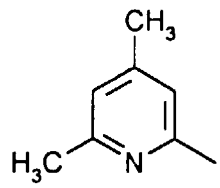, 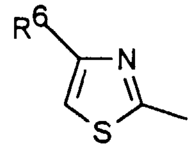,

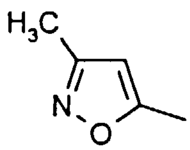, 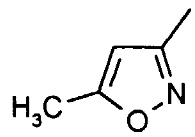, 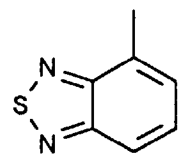, 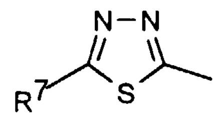

ist,

$R^5$ $C_{1-3}$-Alkyl darstellt,

$R^6$ H oder Phenyl bedeutet und

$R^7$ H, Methyl, tert-Butyl oder Trifluormethyl bedeutet.

Besonders bevorzugte Verbindungen der Formel I sind solche, in denen $R^1$ Ethyl oder Cyclobutyl ist, $R^2$ Phenyl ist oder $R^1$ und $R^2$ zusammen $-(CH_2)_5-$ bedeuten. Insbesondere bevorzugt werden Verbindungen der Formel I, in denen $R^1$ und $R^2$ zusammen $-(CH_2)_5-$ darstellen.

Weitere besonders bevorzugte Verbindungen der Formel I sind solche, in denen $R^3$ H, $C_{1-3}$-Alkyl oder $-CH_2-COOR^5$ ist und $R^5$ Ethyl bedeutet. Insbesondere bevorzugt werden Verbindungen der Formel I, in denen $R^3$ H ist.

Zu besonders bevorzugten Verbindungen der Formel I gehören ferner solche, in denen $R^4$ ein Heteroaromat aus der Gruppe Pyridin-4-yl, Pyridin-3-yl, Thiazol-2-yl, 3-Methyl-isoxazol-5-yl oder 5-Methyl-isoxazol-3-yl ist. Insbesondere bevorzugt werden Verbindungen der Formel I, in denen $R^4$ der Heteroaromat Thiazol-2-yl ist.

Weiterer Erfindungsgegenstand ist ein Verfahren zur Herstellung einer substituierten Imidazolidin-2,4-dion-Verbindung der Formel I, in der

$R^1$ $C_{1-6}$-Alkyl oder $C_{3-6}$-Cycloalkyl bedeutet,

$R^2$ $C_{1-6}$-Alkyl, Phenyl, $-(CH_2)_{1-3}$-Phenyl oder $-(CH_2)_{1-4}-COOR^5$ bedeutet oder

$R^1$ und $R^2$ zusammen$-(CH_2)_{4-6}-$, $-(CH_2)_2-O-(CH_2)_2-$ oder

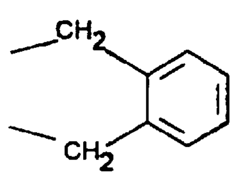

bedeuten,

$R^3$ H, $C_{1-5}$-Alkyl oder $-(CH_2)_{1-4}-COOR^5$ bedeutet,

$R^4$ ein Heteroaromat aus der Gruppe mit den Formeln

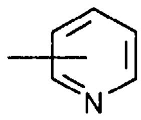 , 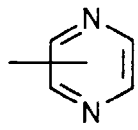 , 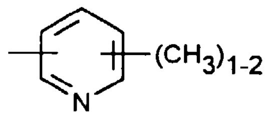 , 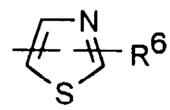 ,

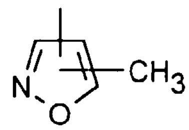 , 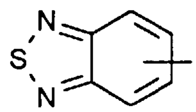 , 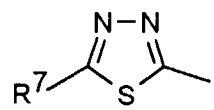

ist,

$R^5$ $C_{1-3}$-Alkyl darstellt,

$R^6$ H, $C_{1-4}$-Alkyl, Phenyl oder Benzyl bedeutet und

$R^7$ H, $C_{1-4}$-Alkyl oder Trifluormethyl bedeutet,

wobei das Verfahren dadurch gekennzeichnet ist, daß man zu einem Amin der Formel II

$$R^4\text{-}NH_2$$

1,1'-Carbonyldiimidazol oder Kohlensäurediphenylester gibt und anschließend mit einer Verbindung der Formel III

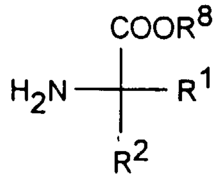

in der $R^8$ H oder $C_{1-3}$-Alkyl darstellt, zu einer Verbindung der Formel I, in der $R^3$ H bedeutet, umsetzt, welche man gewünschtenfalls deprotoniert und anschließend mit einer Verbindung der Formel IV

$$X\text{-}C_{1-5}\text{-Alkyl}$$

oder einer Verbindung der Formel V

$$X\text{-}\ (CH_2)_{1-4}\text{-}COOR^5$$

in denen X Cl, Br oder I bedeutet, zu einer Verbindung der Formel I, in der $R^3$ $C_{1-5}$-Alkyl oder $-(CH_2)_{1-4}$-$COOR^5$ bedeutet, umsetzt.

Die Umsetzung eines Amins der Formel II mit 1,1'-Carbonyldiimidazol oder Kohlensäurediphenylester führt man in an sich bekannter Weise durch [Angew. Chem., 73, 66 (1961)]. Die anschließende Reaktion mit einem Aminosäureester der Formel III zu einer Verbindung der Formel I, in der $R^3$ H ist, führt man vorzugsweise in aprotischen Lösungsmitteln wie Ethern, beispielsweise Diethylether oder Tetrahydrofuran, oder in aromatischen Kohlenwasserstoffen, beispielsweise Toluol, Chlorbenzol oder 1,2-Dichlorbenzol, bei Temperaturen zwischen 20° C und 180° C

durch. Bei dieser Umsetzung kann neben der Verbindung der Formel I, in der $R^3$ H ist, auch das entsprechende Harnstoffderivat der Formel VI

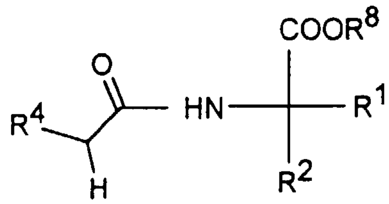

entstehen. Eine Verbindung der Formel VI, bei der $R^8$ H ist, läßt sich durch Reaktion mit Thionylchlorid in eine Verbindung der Formel I, in der $R^3$ H ist, überführen. Eine Verbindung der Formel VI, in der $R^8$ $C_{1-3}$-Alkyl ist, wird vor der Cyclisierung zu einer Verbindung der Formel I, in der $R^3$ H ist, alkalisch verseift oder direkt durch Erhitzen mit Salzsäure in eine Verbindung der Formel I, in der $R^3$ H ist, überführt.

Zur Herstellung einer Verbindung der Formel I, in der $R^3$ $C_{1-5}$-Alkyl oder $-(CH_2)_{1-4}-COOR^5$ bedeutet, deprotoniert man eine Verbindung der Formel I, in der $R^3$ H ist, vorzugsweise mit Natriumhydrid in Dimethylformamid oder Tetrahydrofuran. Die anschließende Umsetzung mit einer Verbindung der Formel IV oder V führt man bei Temperaturen zwischen 20°C und 50°C durch.

Der zur Herstellung einer Verbindung der Formel I benötigte Aminosäureester der Formel III läßt sich durch Veresterung der entsprechenden Aminosäure, beispielsweise mittels Lösungen von Chlorwasserstoff im entsprechenden Alkohol oder durch Erhitzen mit dem entsprechenden Alkohol unter Säurekatalyse, beispielsweise Schwefel- oder Phosphorsäure, herstellen.

Eine weitere Möglichkeit eine Verbindung der Formel III zu erhalten, besteht in der Umsetzung eines Aminosäureesters der Formel VII

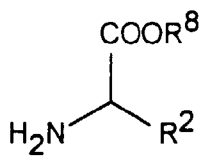

mit Benzaldehyd zu einer Verbindung der Formel VIII,

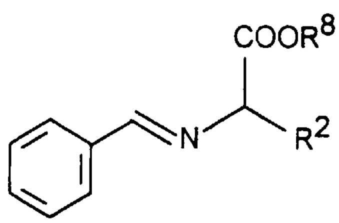

die man nach Deprotonierung mit einer Base, vorzugsweise Lithiumdiisopropylamid, in Ethern oder Kohlenwasserstoffen, beispielsweise Diethylether, Tetrahydrofuran oder Benzol, mit einer Verbindung der Formel IX,

$$X\text{-}R^1$$

in der X Cl, Br oder I bedeutet, alkyliert. Anschließend spaltet man unter Einwirkung von Säuren die Benzylidengruppe ab.

Die erfindungsgemäßen Verbindungen sind toxologisch unbedenklich und eignen sich deshalb als pharmazeutische Wirkstoffe. Dementsprechend ist Erfindungsgegenstand auch die Verwendung einer substituierten Imidazolidin-2,4-dion-Verbindung der Formel I als Wirkstoff in Arzneimitteln, vorzugsweise als Immunmodulatoren oder in

Arzneimitteln mit antivaskulitischer Wirkung.

Erfindungsgemäße Arzneimittel enthalten neben mindestens einer substituierten Imidazolidin-2,4-dion-Verbindung der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trokkenzubereitungen sowie Sprays. Erfindungsgemäße Verbindungen in einem Depot in gelöster Form, einer Trägerfolie oder einem Pflaster, gegebenfalls unter Zusatz von die Hautpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 1 bis 150 mg pro kg wenigstens einer substituierten Imidazolidin-2,4-dion-Verbindung der Formel I appliziert.

**Beispiele**

Als stationäre Phase für die Säulenchromatographie wurde Kieselgel 60 (0,040 - 0,0063 mm) der Firma E. Merck, Darmstadt, eingesetzt.

Die Mischungsverhältnisse der Elutionsmittel für die chromatographischen Methoden sind stets in Volumen/Volumen angegeben.

Racemattrennungen wurden auf einer Chiracel OD Säule der Firma Daicel Chemical Industries, LTD durchgeführt.

Schmp. bedeutet Schmelzpunkt, Bsp. Beispiel, RT Raumtemperatur und d. Th. der Theorie

**Herstellung erfindungsgemäßer Verbindungen**

**Beispiel 1A**

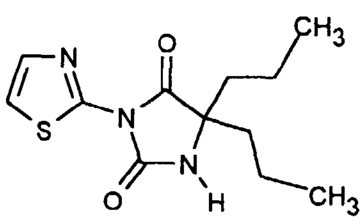

**5,5-Dipropyl-3-thiazol-2-yl-imidazolidin-2,4-dion**

Stufe 1:

2-Amino-pentansäureethylester

Eine Suspension von 11,72 g DL-Norvalin in 90 ml Ethanol wurde mit 3,6 ml konzentierter Schwefelsäure versetzt und das Gemisch acht Tage unter Rückfluß gekocht. Es bildete sich eine klare Lösung, aus welcher Ethanol nach dem Abkühlen destillativ entfernt wurde. Der Rückstand wurde in 200 ml destilliertem Wasser aufgenommen und ein pH-Wert zwischen 10 und 12 durch Zugabe von Kaliumcarbonat eingestellt. Anschließend wurde dreimal mit je 50 ml Essigsäureethylester extrahiert, einmal mit 50 ml einer gesättigten Natriumchloridlösung gewaschen und über Natriumsulfat getrocknet. Nach destillativer Entfernung der Lösungsmittel wurden 11,93 g 2-Amino-pentansäureethylester (82 % d. Th.) in Form eines gelblichen Öls erhalten.

Stufe 2:

2-(Benzyliden-amino)-pentansäureethylester

Eine Lösung von 11,90 g des Produkts der Stufe 1 in 150 ml Diethylether wurde nacheinander mit 8,3 ml Benzaldehyd, 23 ml Triethylamin und 7,0 g wasserfreiem Magnesiumsulfat versetzt. Das Gemisch wurde 24 Stunden bei Raumtemperatur gerührt, anschließend filtriert und mit Dieethylether gewaschen. Nach destillativer Entfernung des Lösungsmittels wurden 18,40 g 2-(Benzylidenamino)-pentansäureethylester (96 % d. Th.) in Form einer gelblichen viskosen Masse erhalten.

Stufe 3:

2-Amino-2-propyl-pentansäureethylester

Eine Lösung von 10,4 ml Diisopropylamin in 200 ml Tetrahydrofuran wurde bei 0° C unter Rühren und unter Überleiten von trockenem Stickstoff tropfenweise mit 49 ml einer 1,6 molaren Lösung von n-Butyllithium in n-Hexan versetzt. Nach Abkühlen auf -78° C wurde eine Lösung von 18,31 g des Produktes aus Stufe 2 in 80 ml Tetrahydrofuran zugetropft. Der gesamte Reaktionsansatz wurde 30 Minuten gerührt und anschließend tropfenweise eine Lösung von 8,8 ml 1-Iodpropan in 40 ml Tetrahydrofuran zugegeben. Der Reaktionsansatz wurde 16 Stunden gerührt, wobei die Temperatur langsam auf 20° C anstieg. Die Lösungsmittel wurden abdestilliert. Der erhaltene orangefarbene Rückstand wurde in 500 ml 1 N Salzsäure aufgenommen. Nach einer Stunde Rühren bei 20° C wurde dreimal mit je 100 ml Diethylether extrahiert. Die salzsaure Phase wurde mit Kaliumhydroxid auf einen pH-Wert zwischen 10 und 12 eingestellt und danach dreimal mit je 100 ml Diethylether extrahiert. Die Extrakte wurden vereinigt, zweimal mit einer gesättigten Natriumchloridlösung gewaschen und über Natriumsulfat getrocknet. Das nach destillativer Entfernung des Lösungsmittels erhaltene Rohprodukt wurde über eine Kieselgelsäule mit Essigsäureethylester gereinigt. Es wurden 9,84 g 2-Amino-2-propyl-pentansäureethylester (67 % d. Th.) in Form eines leicht gefärbten Öls erhalten.

Stufe 4:

2-Propyl-2-(3-thiazol-2-yl-ureido)-pentansäureethylester

Eine Lösung von 5,40 g 2-Aminothiazol in 150 ml Tetrahydrofuran wurde bei 20° C mit 8,75 g 1,1'-Carbonyl-di-imidazol versetzt und das Gemisch 30 Minuten gerührt. Anschließend wurde innerhalb von 20 Minuten auf eine Badtemperatur zwischen 55 und 60° C erwärmt und eine Lösung von 9,80 g des Produktes aus Stufe 3 in 30 ml Tetrahydrofuran zugetropft. Es entstand eine klare rotbraune Lösung, die 60 Stunden bei einer Temperatur zwischen 55° C und 60° C gerührt wurde. Nach destillativer Entfernung des Lösungsmittels wurde der Rückstand über eine Kieselgelsäule mit Essigsäureethylester gereinigt. Es wurden 10,20 g 2-Propyl-2-(3-thiazol-2-yl-ureido)-pentansäureethylester (62 % d. Th.) in Form eines gelblichen Öls erhalten.

Stufe 5:

2-Propyl-2-(3-thiazol-2-yl-ureido)-pentansäure

10,03 g des Produkts aus Stufe 4 wurden unter Rühren in 200 ml halbkonzentrierter Natronlauge bei einer Temperatur von 20° C gelöst. Anschließend wurde ein pH-Wert von 4 mit konzentrierter Salzsäure eingestellt und dreimal mit je 50 ml Dichlormethan extrahiert. Die Extrakte wurden einmal mit einer gesättigten Natriumchloridlösung gewaschen und über Natriumsulfat getrocknet. Nach destillativer Entfernung des Lösungsmittels wurden 8,48 g 2-Propyl-2-(3-thiazol-2-yl-ureido)-pentansäure (93 % d. Th.) in Form weißer Kristalle (Schmp. 154 - 155° C) erhalten.

Stufe 6:

5,5-Dipropyl-3-thiazol-2-yl-imidazolidin-2,4-dion

8,28 g des Produkts aus Stufe 5 wurden mit 20 ml Thionylchlorid versetzt. Das Gemisch wurde 18 Stunden bei 20° C gerührt. Anschließend wurde Eis zur Zersetzung zugegeben, mit Kaliumcarbonat ein alkalischer pH-Wert eingestellt und dreimal mit je 20 ml Dichlormethan extrahiert. Nach Waschen der Extrakte mit gesättigter Natriumchlorid-Lösung und Trocknen über Natriumsulfat wurde das Lösungsmittel destillativ entfernt. Der erhaltene Rückstand wurde über eine Kieselgelsäule mit Essigsäureethylester gereinigt. Es wurden 5,50 g 5,5-Dipropyl-3-thiazol-2-yl-imidazolidin-2,4-dion (71 % d. Th.) in Form weißer Kristalle (Schmp. 127 - 128° C) erhalten.

**Beispiel 1B**

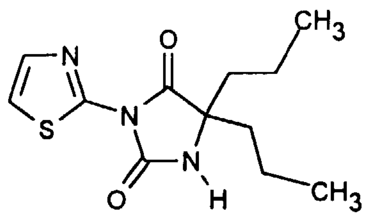

5,5-Dipropyl-3-thiazol-2-yl-imidazolidin-2,4-dion

1,71 g 2-Propyl-2-(3-thiazol-2-yl-ureido)-pentansäureethylester (Produkt aus Beispiel 1A, Stufe 4) wurden mit 30 ml 30 %iger Salzsäure versetzt. Das Gemisch wurde drei Stunden unter Rückfluß gekocht. Nach dem Abkühlen wurde mit Kaliumcarbonat ein alkalischer pH-Wert eingestellt, dreimal mit Essigsäureethylester extrahiert, zweimal mit gesättigter Kochsalzlösung gewaschen und über Natriumsulfat getrocknet. Das durch anschließende destillative Entfernung des Lösungsmittels erhaltene Rohprodukt wurde über eine Kieselgelsäule mit Essigsäureethylester gereinigt. Es wurden 0,62 g 5,5-Di-propyl-3-thiazol-2-yl-imidazolidin-2,4-dion (42 % d. Th.) erhalten.

**Beispiel 2**

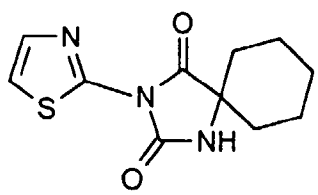

3-Thiazol-2-yl-1,3-diaza-spiro[4.5]decan-2,4-dion

Stufe 1:

1-Amino-1-cyclohexancarbonsäureethylester

Unter den in Beispiel 1A Stufe 1 beschriebenen Bedingungen erhielt man aus 100 g 1-Amino-1-cyclohexancarbonsäure, Hydrochlorid, 500 ml Ethanol und 20 ml konzentrierter Schwefelsäure nach Reinigung des Rohproduktes über eine Kieselgelsäule mit Essigsäureethylester/Methanol = 5/1 75,5 g 1-Amino-1-cyclohexancarbonsäureethylester (80 % d. Th.) in Form eines leicht gelben Öls.

Stufe 2:

3-Thiazol-2-yl-1,3-diaza-spiro[4.5]decan-2,4-dion

44,4 g 2-Aminothiazol, 71,9 g 1,1'-Carbonyl-diimidazol und 73,7 g des Produktes aus Stufe 1 wurden entsprechend den in Beispiel 1A, Stufe 4 beschrieben Bedingungen umgesetzt. Das erhaltene Produktgemisch wurde über eine Kieselgelsäule mit Essigsäureethylester gereinigt. Es wurden 71,8 g 3-Thiazol-2-yl-1,3-diaza-spiro[4.5]decan-2,4-dion (66 % d. Th.) in Form weißer Kristalle (Schmp. 213 - 215° C) erhalten.

**Beispiel 3**

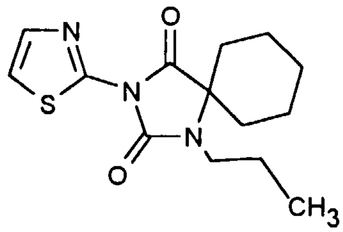

**1-Propyl-3-thiazol-2-yl-1,3-diaza-spiro[4.5]decan-2,4-dion**

5,05 g des Produktes aus Beispiel 2 Stufe 2 wurden in 20 ml Dimethylformamid gelöst. Anschließend wurden 1,10 g Natriumhydrid (50%-ige Suspension in Mineralöl) unter Rühren bei 20° C portionsweise zugegeben. Nach einer Stunde Rühren wurden 4 ml 1-Iodpropan zugefügt. Es wurde weitere drei Stunden gerührt. Anschließend wurde mit 100 ml destilliertem Wasser verdünnt, dreimal mit je 30 ml Essigsäureethylester extrahiert, mit gesättigter Natriumchloridlösung gewaschen und über Natriumsulfat getrocknet. Nach destillativer Entfernung des Lösungsmittels wurde der Rückstand über eine Kieselgelsäule mit Essigsäureethylester/n-Hexan = 8/5 gereinigt. Es wurden 3,95 g 1-Propyl-3-thiazol-2-yl-1,3-diaza-spiro[4.5]decan-2,4-dion (67 % d. Th.) in Form weißer Kristalle (Schmp. 135 - 138° C) erhalten.

**Beispiel 4**

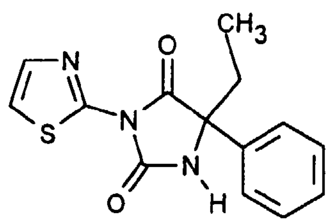

**5-Ethyl-5-phenyl-3-thiazol-2-yl-imidazolidin-2,4-dion**

Stufe 1:

2-Amino-2-phenyl-buttersäureethylester

10,0 g 2-Amino-2-phenyl-buttersäure wurden mit 140 ml einer ethanolischen Lösung von Chlorwasserstoff (10 % HCl) 10 Tage bei 30° C gerührt. Anschließend wurde Ethanol abdestilliert, der Rückstand in 200 ml destilliertem Wasser aufgenommen und mit Kaliumcarbonat ein alkalischer pH-Wert eingestellt. Nach dreimaliger Extraktion mit Essigsäureethylester, Trocknen der Extrakte über Natriumsulfat und destillativer Entfernung des Lösungsmittels wurde über eine Kieselgelsäule mit Essigsäureethylester gereinigt. Es wurden 6,93 g 2-Amino-2-phenyl-buttersäureethylester (62 % d. Th.) in Form eines gelblichen Öls erhalten.

2.Stufe

5-Ethyl-5-phenyl-3-thiazol-2-yl-imidazolidin-2,4-dion

2,12 g 2-Aminothiazol, 3,26 g 1,1'-Carbonyl-diimidazol und 4,16 g des Produkts aus Stufe 1 wurden unter den in Beispiel 1A, Stufe 4 beschrieben Bedingungen umgesetzt. Nach Reinigung des Rohgemisches über eine Kieselgelsäule mit Essigsäureethylester wurden 3,90 g 5-Ethyl-5-phenyl-3-thiazol-2-yl-imidazolidin-2,4-dion (68 % d. Th.) in Form weißer Kristalle (Schmp. 150 - 152° C)erhalten.

**Beispiel 5**

**(+)- und (-) -5-Ethyl-5-phenyl-3-thiazol-2-yl-imidazolidin-2,4-dion**

Die beiden Enantiomeren wurden durch Trennung des Racemates aus Beispiel 4 auf einer chiralen HPLC-Säule erhalten (Fließmittel: n-Hexan/2-Propanol = 1/1; stationäre Phase: Cellulose-tris-3,5-dimethylphenyl-carbamat).

**Beispiel 6**

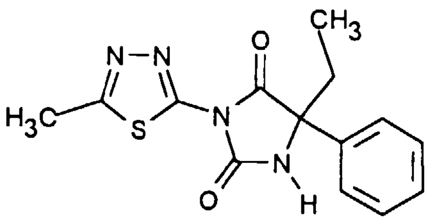

**5-Ethyl-3-(5-methyl[1.3.4]thiadiazol-2-yl)-5-phenylimidazolidin-2,4-dion**

In einer Stickstoffatmosphäre und unter Ausschluß von Feuchtigkeit wurden 2,30 g 2-Amino-5-methyl-1,3,4-thiadiazol bei Raumtemperatur in 40 ml trockenem Tetrahydrofuran gelöst. Anschließend wurden 3,24 g 1,1'-Carbonyldiimidazol zugegeben. Es wurde 30 Minuten bei 50° C gerührt. Zur erhaltenen Suspension wurde eine Lösung von 4,15 g 2-Amino-2-phenyl-buttersäureethylester (Produkt aus Beispiel 3, Stufe 1) in 10 ml trockenem Tetrahydrofuran getropft und 20 Stunden bei 50° C gerührt. Nach destillativer Entfernung des Lösungsmittels wurde der Rückstand aus Ethanol umkristallisiert. Es wurden 3,94 g 5-Ethyl-3-(5-methyl[1.3.4]thiadiazol-2-yl)-5-phenyl-imidazolidin-2,4-dion (58 % d. Th.) in Form weißer Kristalle (Schmp. 223 - 225° C) erhalten.

**Beispiele 7 - 28**

Die in Tabelle 1 aufgeführten Verbindungen wurden aus den entsprechenden Ausgangsverbindungen unter den in den Beispielen 1 - 6 beschriebenen Bedingungen hergestellt.

Tabelle 1:

| Bsp. | Verbindung | $R^1$ | $R^2$ | $R^3$ | $R^4$ | Schmp. [°C] | hergestellt analog Bsp. |
|---|---|---|---|---|---|---|---|
| 7 | 5-Isopropyl-5-phenyl-3-thiazolyl-imidazolidin-2,4-dion | Isopropyl | Phenyl | H | Thiazol-2-yl | 173-175 | 6 |
| 8 | 3-(4-Methyl-2,5-dioxo-1-thiazol-2-yl-imidiazolidin-4-yl)-propionsäure-ethylester | Methyl | 2-Ethoxycarbonyl-ethyl | H | Thiazol-2-yl | 88-90 | 6 |
| 9 | 5-Isobutyl-5-methyl-3-thiazol-2-yl-imidazolidin-2,4-dion | Methyl | Isobutyl | H | Thiazol-2-yl | 138-140 | 6 |
| 11 | 3-Thiazol-2-yl-1,3-diaza-spiro[4.4]benzononan-2,4-dion | Phenylendimethyl | | H | Thiazol-2-yl | 175-177 | 6 |

| Bsp. | Verbindung | $R^1$ | $R^2$ | $R^3$ | $R^4$ | Schmp. [°C] | hergestellt analog Bsp. |
|---|---|---|---|---|---|---|---|
| 12 | 5-Benzyl-5-methyl-3-thiazol-2-yl-imidazolidin-2,4-dion | Methyl | Benzyl | H | Thiazol-2-yl | 197-199 | 1A |
| 13 | 5-Methyl-5-pentyl-3-thiazol-2-yl-imidazolidin-2,4-dion | Methyl | Pentyl | H | Thiazol-2-yl | 109-111 | 1A |
| 14 | 5-Methyl-5-(2-phenylethyl)-3-thiazol-2-yl-imidazolidin-2,4-dion | Methyl | 2-Phenylethyl | H | Thiazol-2-yl | 160-162 | 1A |
| 15 | (2,4-Dioxo-3-thiazol-2-yl-1,3-diazaspiro[4.5]dec-1-yl)-essigsäure-ethylester | Pentamethylen | | Ethoxycarbonyl-methyl | Thiazol-2-yl | 82-88 | 3 |

| Bsp. | Verbindung | $R^1$ | $R^2$ | $R^3$ | $R^4$ | Schmp. [°C] | hergestellt analog Bsp. |
|---|---|---|---|---|---|---|---|
| 16 | 5-Ethyl-5-phenyl-3[1.3.4]thiadiazol-2-yl-imidazolidin-2,4-dion | Ethyl | Phenyl | H | 1,3,4-Thiadiazol-2-yl | 217-220 | 6 |
| 17 | 5-Ethyl-3-(5-methyl-isoxazol-3-yl)-5-phenyl-imidazolidin-2,4-dion | Ethyl | Phenyl | H | 5-Methyl-isoxazol-3-yl | 156-157 | 6 |
| 18 | 3-(5-tert-Butyl[1.3.4]thiadiazol-2-yl)-5-ethyl-5-phenyl-imidazolidin-2,4-dion | Ethyl | Phenyl | H | 5-tert-Butyl-[1.3.4]thiadiazol-2-yl | 145-146 | 6 |
| 19 | 5-Ethyl-5-phenyl-3-pyridin-4-yl-imidazolidin-2,4-dion | Ethyl | Phenyl | H | Pyridin-4-yl | 153-154 | 6 |

| Bsp. | Verbindung | $R^1$ | $R^2$ | $R^3$ | $R^4$ | Schmp. [°C] | hergestellt analog Bsp. |
|---|---|---|---|---|---|---|---|
| 20 | 5-Ethyl-5-phenyl-3-pyrazin-2-yl-imidazolidin-2,4-dion | Ethyl | Phenyl | H | Pyrazin-2-yl | 174-176 | 6 |
| 21 | 5-Ethyl-5-phenyl-3-pyridin-3-yl-imidazolidin-2,4-dion | Ethyl | Phenyl | H | Pyridin-3-yl | 158-160 | 6 |
| 22 | 3-Pyridin-4-yl-1,3-diaza-spiro[4.5]decan-2,4-dion | Pentamethylen | | H | Pyridin-4-yl | 252-254 | 6 |
| 23 | 3-Pyridin-3-yl-1,3-diaza-spiro[4.5]decan-2,4-dion | Pentamethylen | | H | Pyridin-3-yl | 248-249 | 6 |
| 24 | 3-Benzo[1.2.5]thiadiazol-4-yl-5-ethyl-5-phenyl-imidazolidin-2,4-dion | Ethyl | Phenyl | H | Benzo[1.2.5] thiadiazol-4-yl | 154-156 | 6 |

| Bsp. | Verbindung | $R^1$ | $R^2$ | $R^3$ | $R^4$ | Schmp. [°C] | hergestellt analog Bsp. |
|---|---|---|---|---|---|---|---|
| 25 | 5-Ethyl-5-phenyl-3-(5-trifluormethyl[1.3.4]thiadiazol-2-yl)-imidazolidin-2,4-dion | Ethyl | Phenyl | H | 5-Trifluormethyl-[1.3.4]thiadiazol-2-yl | 118-120 | 6 |
| 26 | 3-(4,6-Dimethyl-pyridin-2-yl)-5-ethyl-5-phenyl-imidazolidin-2,4-dion | Ethyl | Phenyl | H | 4,6-Dimethyl-pyridin-2-yl | 141-142 | 1A |
| 27 | 5-Ethyl-5-phenyl-3-(4-phenyl-thiazol-2-yl)-imidazolidin-2,4-dion | Ethyl | Phenyl | H | 4-Phenyl-thiazol-2-yl | 118-120 | 1A |
| 28 | 5-Ethyl-3-(3-methyl-isoxazol-5-yl)-5-phenyl-imidazolidin-2,4-dion | Ethyl | Phenyl | H | 3-Methyl-isoxazol-5-yl | 146-148 | 6 |

Durch Trennung der Racemate aus den Beispielen 17, 18 und 28 unter den in Beispiel 5 beschriebenen Bedingungen wurden die in Tabelle 2 zusammengefaßten Enantiomere in Form von viskosen Ölen erhalten. Bei der Bestimmung des Drehwertes $[\alpha]_D^{RT}$ wurde Methanol als Lösungsmittel verwendet.

Tabelle 2:

| Beispiel | Verbindung | $[\alpha]_D^{RT}$ |
|---|---|---|
| 29 | (+)-5-Ethyl-3-(5-methylisoxazol-3-yl)-5-phenylimidazolidin-2,4-dion | + 36,2° |
| 30 | (-)-5-Ethyl-3-(5-methylisoxazol-3-yl)-5-phenylimidazolidin-2,4-dion | - 36,4° |
| 31 | (+)-3-(5-tert-Butyl [1.3.4]thiadiazol-2-yl)-5-ethyl-5-phenyl-imidazolidin-2,4-dion | + 26,0° |
| 32 | (-)-3-(5-tert-Butyl [1.3.4]thiadiazol-2-yl)-5-ethyl-5-phenyl-imidazolidin-2,4-dion | - 26,1° |
| 33 | (+)-5-Ethyl-3-(3-methylisoxazol-5-yl)-5-phenylimidazolidin-2,4-dion | + 18,5° |
| 34 | (-)-5-Ethyl-3-(3-methylisoxazol-5-yl)-5-phenylimidazolidin-2,4-dion | - 18,2° |

**Pharmakologische Untersuchungen**

Die Freisetzung von TNF-α kann in vitro an humanen mononukleären Zellen des peripheren Blutes (T-Zellen, B-Zellen und Monozyten) nach Stimulation mit Lipopolysaccharid (LPS) untersucht werden (siehe nachfolgend unter 1.). LPS ist ein Bestandteil der bakteriellen Zellwand und stimuliert Monozyten und Makrophagen.

Neben der Stimulation mit LPS kann die Freisetzung von TNF-α auch durch Stimulation von humanen mononukleären Zellen des peripheren Blutes mit T-zellspezifischen, monoklonalen Antikörpern gegen Aktivierungsantigene (antiCD2/ antiCD28) oder dem bakteriellen Superantigen Toxic Shock Syndrome Toxin-1 (TSST-1) provoziert werden. Abgesehen von der TNF-α Freisetzung führen diese Stimulanzien unter anderem auch zur Bildung von Interleukin-2 (IL-2). Verbindungen, die eine generelle Immunsuppression zur Folge haben, hemmen sowohl die TNF-α als auch die IL-2 Freisetzung. Verbindungen hingegen, die nicht zu einer Blockierung der zellulären Immunabwehr führen, sollten die LPS-stimulierte TNF-α Freisetzung gut inhibieren, bei der T-zellspezifischen stimulierten Freisetzung von IL-2 aber nur eine geringe Hemmung induzieren (siehe nachfolgend unter 2.).

**1. Wirkung auf die TNF-α Freisetzung (in vitro)**

Die hemmende Wirkung der erfindungsgemäßen Verbindungen in bezug auf die Freisetzung von TNF-α wurde in einem in vitro-Test mit mononukleären Zellen getestet.

Mononukleäre Zellen wurden aus dem heparinisierten Blut von mindestens drei freiwilligen Spendern gewonnen. Hierzu wurden je 20 ml Blut in bekannter Weise über einen Ficoll-Paque-Gradienten getrennt. Die Zellen wurden geerntet und dreimal mit einem Zellkulturmedium gewaschen. Das verwendete Zellkulturmedium bestand aus RPMI 1640 Medium mit 2 mM Glutamin (Life Technologies, Eggenstein) supplementiert mit 10 % fötalem Kälberserum (Life Technologies), 50 µg/ml Streptomycin (Sigma, Deisenhofen), 50 IU/ml Penicillin (Sigma) und 100 µM ß-Mercaptoethanol (Merck, Darmstadt). Die mononukleären Zellen wurden anschließend in 15 ml Zellkulturmedium aufgenommen und in 1 ml Ansätzen in sterilen 24-Loch-Inkubationsplatten (Sigma) aufgeteilt. Den 1-ml-Ansätzen, die man als Kontrollansatz benutzte, wurden jeweils 1 µl Dimethylsulfoxid (DMSO, Merck) zugesetzt. Den Testansätzen wurde 1 µl einer Lösung einer erfindungsgemäßen Verbindung (in DMSO; Endkonzentrationen im Test: 0,5; 5; 12,5 und 50 µg/ml) zugegeben. Die Ansätze wurden eine Stunde im $CO_2$-Brutschrank (5 % $CO_2$, 90 % Luftfeuchtigkeit) inkubiert. Anschließend wurden, mit Ausnahme der Kontrollansätze, jeweils 2,5 µg LPS (von E. coli 0127:B8; Sigma, Deisenhofen) als Stimulans zugefügt. Die Inkubation der Ansätze wurde 20 Stunden fortgesetzt. Die Konzentration von TNF-α in den Zellkulturüberständen der Ansätze wurde im Anschluß an die Inkubation mit ELISA-Tests (Boehringer-Mannheim) bestimmt. Aus den Meßwerten der Kontrollansätze und den mit den erfindungsgemäßen Verbindungen inkubierten Testansätzen wurde die Stärke der Hemmung der TNF-α Freisetzung berechnet. Mit Hilfe einer Regressionsgerade wurden die Konzentrationen errechnet, die zu einer 50 %igen Hemmung der TNF-α Freisetzung führten ($IC_{50}$-Werte).

Alle eingesetzten erfindungsgemäßen Verbindungen zeigten eine ausgeprägte inhibitorische Wirkung auf die LPS-stimulierte Freisetzung von TNF-α. Die Ergebnisse sind in der nachfolgenden Tabelle 3 dargestellt.

Tabelle 3:

| Wirkung auf die LPS-stimulierte TNF-α Freisetzung (Mittelwert und Standardabweichung) | | |
|---|---|---|
| **erfindungsgemäße Verbindung hergestellt nach Beispiel** | **Hemmung der TNF-α-Freisetzung in % bei einer Endkonzentration von 50 μg/ml im Test** | **$IC_{50}$ [μg/ml]** |
| 1 | 83 ± 8 | |
| 2 | 66 ± 18 | 31 |
| 3 | 80 ± 12 | |
| 4 | 90 ± 3 | 8 |
| 5 (+)-Isomer | 93 ± 6 | 4 |
| 5 (-)-Isomer | 74 ± 16 | 10 |
| 6 | 74 ± 19 | |
| 8 | 48 ± 14 | |
| 10 | 76 ± 9 | 9 |
| 11 | 70 ± 16 | |
| 13 | 73 ± 13 | 24 |
| 14 | 74 ± 6 | |
| 15 | 78 ± 14 | |
| 16 | 69 ± 8 | |
| 18 | 65 ± 29 | |
| 19 | 92 ± 4 | <1 |
| 20 | 87 ± 3 | 5 |
| 22 | 80 ± 7 | |
| 23 | 68 ± 6 | |
| 24 | 89 ± 4 | |
| 25 | 49 ± 13 | |
| 26 | 71 ± 8 | |
| 27 | 73 ± 20 | |
| 29 | 87 ± 4 | 7 |
| 30 | 61 ± 6 | |
| 31 | 68 ± 11 | |
| 33 | 92 ± 4 | 6 |

**2. Wirkung auf die zelluläre Immunabwehr (in vitro)**

In der nachstehend beschriebenen in vitro Testreihe wurden unterschiedlich stimulierte mononukleäre Zellen eingesetzt, um die Wirkung der erfindungsgemäßen Verbindungen auf die zelluläre Immunabwehr zu untersuchen.

Erfindungsgemäße Verbindungen wurden hinsichtlich ihrer Wirkung auf die TNF-α und IL-2 Freisetzung überprüft. Die Durchführung der Versuche erfolgte entsprechend den unter 1. beschriebenen Bedingungen. Die Stimulanzien wurden für jede Testreihe variiert. Als Stimulanzien wurden entweder monoklonale Antikörper antiCD2/anti-CD28, Superantigen TSST-1 oder LPS benutzt.

Folgende Endkonzentrationen der Stimulanzien wurden eingestellt:

antiCD2/antiCD28: 100 ng/ml AICD2.M1; 100 ng/ml AICD2.M2 (monoklonale Antikörper, beide gegen CD2 gerichtet, Herkunft Deutsches Krebsforschungszentrum, Prof. Dr. Meuer, Heidelberg);

| | |
|---|---|
| | 0,1 % % (vol/vol) anti CD28 Ascites Flüssigkeit (CLB, Amsterdam) |
| Superantigen: | 0,1 µg/ml TSST-1 (Sigma, Deisenhofen) |
| LPS: | 2,5 µg LPS (von E. coli 0127:B8; Sigma, Deisenhofen) |

Die erfindungsgemäßen Verbindungen wurden in Konzentrationen (siehe Tabelle 4, Spalte 2) eingesetzt, die eine 60 - 90 %ige Hemmung der LPS-induzierten TNF-α Freisetzung bewirkten.

Bei den mit dem Antikörpergemisch antiCD2/antiCD28 oder mit dem Superantigen TSST-1 stimulierten Testansätzen wurde nach Versuchsende in den Zellkulturüberständen die IL-2 Konzentration mit ELISAs (Boehringer-Mannheim) bestimmt.

Die eingesetzten erfindungsgemäßen Verbindungen bewirkten keinen generellen immunsuppressiven Effekt, da im Gegensatz zu Dexamethason nur eine relativ geringe Inhibition der IL-2 Freisetzung induziert wurde.

Die Ergebnisse sind in Tabelle 4 zusammengefaßt:

Tabelle 4:

Wirkung auf die Freisetzung von TNF-α und IL-2 bei unterschiedlichen Stimulationsbedingungen (Mittelwert und Standardabweichung)

| erfindungsgemäße Verbindung hergestellt nach Beispiel | eingesetzte Konzentration im Test | Hemmung TNF-α LPS stimuliert [%] | Hemmung IL-2 antiCD2/ antiCD28 stimuliert [%] | Hemmung IL-2 TSST-1 stimuliert [%] |
|---|---|---|---|---|
| Dexamethason | [ 5,0 µg/ml] | 86,2 ± 1,5 | 58,3 ± 21,1 | 83,9 ± 14,5 |
| 2 | [50,0 µg/ml] | 66,0 ± 18,1 | 7,3 ± 29,1 | 12,4 ± 15,7 |
| 4 | [50,0 µg/ml] | 92,2 ± 3,1 | 21,2 ± 27,3 | 55,4 ± 8,5 |
| 10 | [12,5 µg/ml] | 61,1 ± 13,9 | 13,2 ± 19,6 | 33,9 ± 6,4 |
| 19 | [ 5,0 µg/ml] | 73,1 ± 8,9 | 34,8 ± 3,1 | 35,1 ± 2,3 |

### 3. Antivaskulitische Wirkung im Tiermodell

Für die in-vivo Charakterisierung der antivaskulitischen Wirkungen erfindungsgemäßer Verbindungen der Formel I wurde ein zweiphasiges Modell, das in seinen Ursprüngen auf die lokale Shwartzman-Reaktion zurückgeht, eingesetzt [Exp. Toxic. Pathol., 47, 167, (1995)]. Mit diesem Tiermodell ist eine Inhibition der Endothelpermeabilität, die nicht nur auf die Hemmung der TNF-α Freisetzung zurückzuführen ist, nachweisbar. Über den quantifizierten Parameter der Endothelpermeabilität hinaus ist qualitativ die weitgehende Reduktion oder das Ausblieben der für die Shwartzman-Reaktion kennzeichnende Gewebedestruktion feststellbar.

Männliche NMRI-Mäuse wurden unter Kurznarkose dorsal enthaart. An symmetrischen Stellen wurden beiderseits 100 µg Lipopolysaccharid (Salmonella typhosa; Sigma, Deisenhofen) oder, als Kontrolle, physiologische Kochsalzlösung intradermal injiziert. 24 Stunden später wurde über die Schwanzvene Evans Blau (Merck, Darmstadt) in einer Konzentration von 1 ml/kg appliziert. Anschließend wurde eine subkutane Injektion von rekombinantem, murinem TNF-α (133 ng) unter die beiden LPS-sensitivierten Hautabschnitte vorgenommen. Vier Stunden nach der TNF-α Provokation wurden die Mäuse euthanasiert und die Hautabschnitte in definiertem Umfang ausgestanzt. Der Gehalt an Evans Blau in den Hautproben wurde durch eine photometrische Extinktionsbestimmung bei 623 nm, die sich an eine 18-stündige Extraktion in Formamid bei 60° C anschloß, gemessen.

Die erfindungsgemäßen Verbindungen wurden in einer wäßrigen 1%igen Carboxylmethylcelluloselösung suspendiert und intraperitoneal oder oral appliziert. Die Gabe der erfindungsgemäßen Verbindungen erfolgte bei intraperitonealer Gabe jeweils 10 Minuten vor Applikation des LPS oder des TNF-α, bei oraler Gabe 30 Minuten vor diesen Stimuli. Eine weitere Gabe der erfindungsgemäßen Verbindungen erfolgte während der Präparationsphase 8 Stunden nach der LPS-Injektion. Die Dosierungen betrugen 5 - 400 mg/kg. Zur Kontrolle wurden auch Tiere mit NaCl anstelle von LPS vorbehandelt.

In Tabelle 5 sind maximale Hemmeffekte in % bei LPS-präparierten Tieren, die mit erfindungsgemäßen Verbindungen behandelt wurden, im Vergleich mit NaCl-präparierten Tieren, die mit erfindungsgemäßen Verbindungen behandelt wurden, dargestellt. Die Prozentangaben stellen Mittelwerte von ≥ 10 Tieren pro Gruppe dar.

Die erfindungsgemäßen Verbindungen weisen eine antivaskulitische Wirkung auf, die durch den Nachweis der Inhibition der Endothelpermeabilität quantifiziert werden konnte. Die Ergenisse sind in Tabelle 5 zusammengefaßt.

Tabelle 5:

| Inhibition der Endothelpermeabilität (Evans Blau Extraktion) | | |
|---|---|---|
| **erfindungsgemäße Verbindung hergestellt nach Bsp.** | **eingesetzte Konzentration im Test [mg/kg]** | **Maximale Hemmung der Evans Blau Extravasation** |
| 2 | 3 x 50 | 67 % |
| 4 | 3 x 100 | 48 % |
| 5 (+)-Isomer | 3 x 50 | 38 % |
| 5 (-)-Isomer | 3 x 100 | 58 % |

## Patentansprüche

1. Substituierte Imidazolidin-2,4-dion-Verbindungen der Formel I

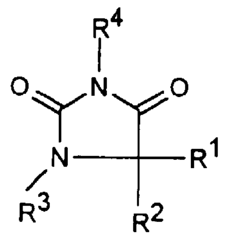

in der

$R^1$ $C_{1-6}$-Alkyl oder $C_{3-6}$-Cycloalkyl bedeutet,

$R^2$ $C_{1-6}$-Alkyl, Phenyl, $-(CH_2)_{1-3}$-Phenyl oder $-(CH_2)_{1-4}$-$COOR^5$ bedeutet

oder

$R^1$ und $R^2$ zusammen $-(CH_2)_{4-6}$-, $-(CH_2)_2$-O-$(CH_2)_2$-oder

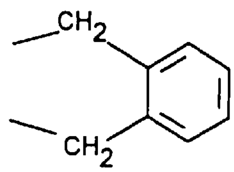

bedeuten,

$R^3$ H, $C_{1-5}$-Alkyl oder $-(CH_2)_{1-4}$-$COOR^5$ bedeutet,

$R^4$ ein Heteroaromat aus der Gruppe mit den Formeln

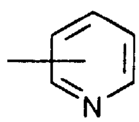 , 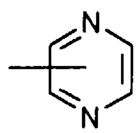 , 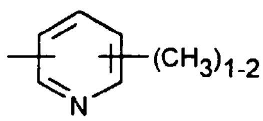 , 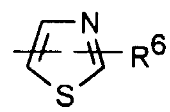 ,

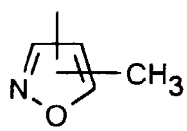 , 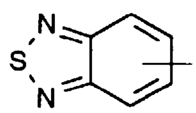 , 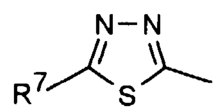

ist,

$R^5$ $C_{1-3}$-Alkyl darstellt,

$R^6$ H, $C_{1-4}$-Alkyl, Phenyl oder Benzyl bedeutet und

$R^7$ H, $C_{1-4}$-Alkyl oder Trifluormethyl bedeutet.

**2.** Substituierte Imidazolidin-2,4-dion-Verbindungen der Formel I gemäß Anspruch 1, dadurch gekennzeichnet, daß

$R^1$ $C_{1-4}$-Alkyl oder $C_{3-4}$-Cycloalkyl bedeutet,

$R^2$ $C_{3-6}$-Alkyl, Phenyl, $-(CH_2)_{1-2}$-Phenyl oder $-(CH_2)_{1-2}$-$COOR^5$ bedeutet,

oder

$R^1$ und $R^2$ zusammen $-(CH_2)_5$- oder

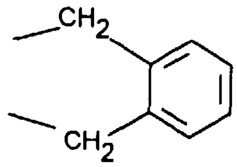

bedeuten,

$R^3$ H, $C_{1-4}$-Alkyl oder $-(CH_2)_{1-2}$-$COOR^5$ bedeutet,

$R^4$ ein Heteroaromat aus der Gruppe mit den Formeln

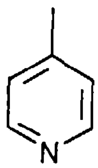 , 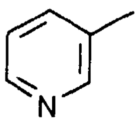 , 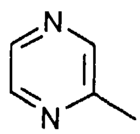 , 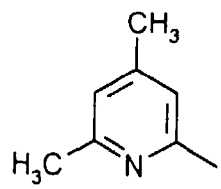 , 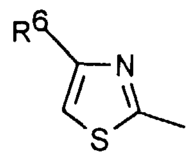 ,

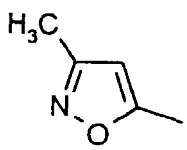 , 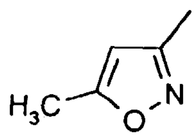 , 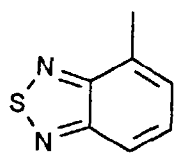 , 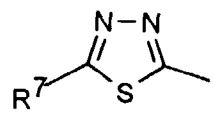

ist,

$R^5$ $C_{1-3}$-Alkyl darstellt,

$R^6$ H oder Phenyl bedeutet und

$R^7$ H, Methyl, tert-Butyl oder Trifluormethyl bedeutet.

**3.** Substituierte Imidazolidin-2,4-dion-Verbindungen der Formel I gemäß Anspruch 1 oder 2, dadurch gekennzeichnet, daß

$R^1$ Ethyl oder Cyclobutyl bedeutet,

$R^2$ Phenyl ist oder

$R^1$ und $R^2$ zusammen $-(CH_2)_5-$ bedeuten.

**4.** Substituierte Imidazolidin-2,4-dion-Verbindungen der Formel I gemäß einem der Ansprüche 1 bis 3, dadurch gekennzeichnet, daß

$R^1$ und $R^2$ zusammen $-(CH_2)_5-$ bedeuten.

**5.** Substituierte Imidazolidin-2,4-dion-Verbindungen der Formel I gemäß einem der Ansprüche 1 bis 4, dadurch gekennzeichnet, daß

$R^3$ H, $C_{1-3}$-Alkyl oder $-CH_2$-$COOR^5$ bedeutet und

$R^5$ Ethyl ist.

**6.** Substituierte Imidazolidin-2,4-dion-Verbindungen der Formel I gemäß Anspruch 5, dadurch gekennzeichnet, daß

$R^3$ H bedeutet.

**7.** Substituierte Imidazolidin-2,4-dion-Verbindungen der Formel I gemäß einem der Ansprüche 1 bis 6, dadurch gekennzeichnet, daß

$R^4$ Pyridin-4-yl, Pyridin-3-yl, Thiazol-2-yl, 3-Methyl-isoxazol-5-yl oder 5-Methyl-isoxazol-3-yl bedeutet.

**8.** Substituierte Imidazolidin-2,4-dion-Verbindungen der Formel I gemäß Anspruch 7, dadurch gekennzeichnet, daß

$R^4$ Thiazol-2-yl ist.

**9.** Verfahren zur Herstellung einer substituierten Imidazolidin-2,4-dion-Verbindung der Formel I gemäß Anspruch 1

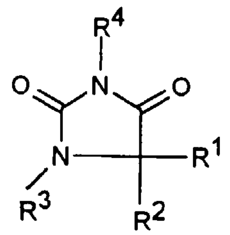

dadurch gekennzeichnet, daß
man zu einem Amin der Formel II

$$R^4\text{-}NH_2$$

1,1'-Carbonyldiimidazol oder Kohlensäurediphenylester gibt und anschließend mit einer Verbindung der Formel III

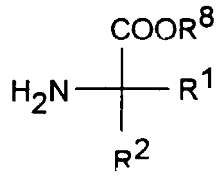

in der $R^8$ H oder $C_{1-3}$-Alkyl darstellt,
zu einer Verbindung der Formel I, in der $R^3$ H bedeutet, umsetzt,
die man gewünschtenfalls deprotoniert und anschließend mit einer Verbindung der Formel IV

$$X\text{-}C_{1-5}\text{-Alkyl}$$

oder mit einer Verbindung der Formel V

$$X\text{-}(CH_2)_{1-4}\text{-}COOR^5$$

in denen X Cl, Br oder I bedeutet, zu einer Verbindung der Formel I, in der $R^3$ $C_{1-5}$-Alkyl oder $-(CH_2)_{1-4}$-$COOR^5$ bedeutet, umsetzt.

**10.** Substituierte Imidazolidin-2,4-dion-Verbindungen der Formel I gemäß Anspruch 1 zur Verwendung als Wirkstoff in einem Arzneimittel.

**11.** Substituierte Imidazolidin-2,4-dion-Verbindungen der Formel I gemäß Anspruch 1 zur Verwendung nach Anspruch 10, dadurch gekennzeichnet, daß das Arzneimittel ein Immunmodulator ist.

**12.** Substituierte Imidazolidin-2,4-dion-Verbindungen der Formel I gemäß Anspruch 1 zur Verwendung nach Anspruch 10, dadurch gekennzeichnet, daß das Arzneimittel eine antivaskulitische Wirkung hat.

**Claims**

**1.** Substituted 2,4-imidazolidinedione compounds of the formula I

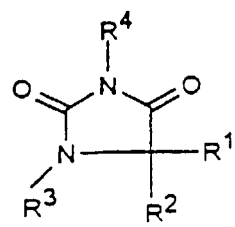

in which

$R^1$ means $C_{1-6}$ alkyl or $C_{3-6}$ cycloalkyl,

$R^2$ means $C_{1-6}$ alkyl, phenyl, $-(CH_2)_{1-3}$-phenyl or $-(CH_2)_{1-4}-COOR^5$

or

$R^1$ and $R^2$ together mean $-(CH_2)_{4-6}-$ , $-(CH_2)_2-O-(CH_2)_2-$ or

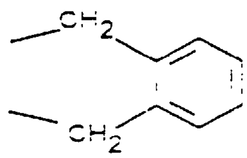

$R^3$ means H, $C_{1-5}$ alkyl or $-(CH_2)_{1-4}-COOR^5$,

$R^4$ is a heteroaromatic selected from the group of the formulae

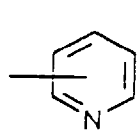 , 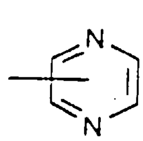 , 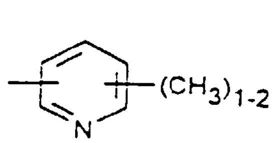 , 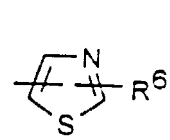 ,

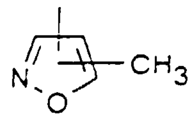 , 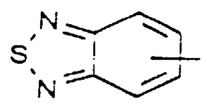 , 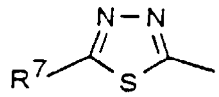

$R^5$ denotes $C_{1-3}$ alkyl,

$R^6$ means H, $C_{1-4}$ alkyl, phenyl or benzyl and

$R^7$ means H, $C_{1-4}$ alkyl or trifluoromethyl.

**2.** Substituted 2,4-imidazolidinedione compounds of the formula I according to claim 1, characterised in that

$R^1$ means $C_{1-4}$ alkyl or $C_{3-4}$ cycloalkyl,

$R^2$ $C_{3-6}$ alkyl, phenyl, $(CH_2)_{1-2}$-phenyl or $-(CH_2)_{1-2}-COOR^5$, or

$R^1$ and $R^2$ together mean $-(CH_2)_5-$ or

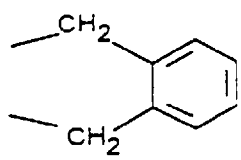

$R^3$ means H, $C_{1-4}$ alkyl or $-(CH_2)_{1-2}-COOR^5$,

$R^4$ is a heteroaromatic selected from the group of the formulae

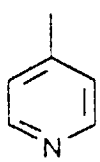 , 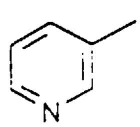 , 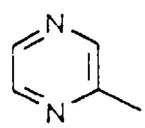 , 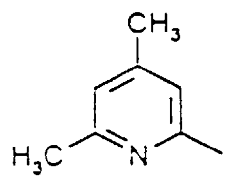 , 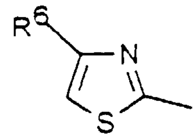 ,

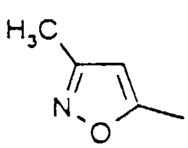 , 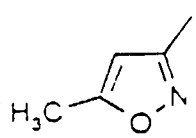 , 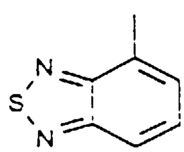 , 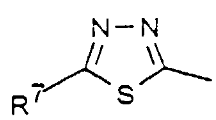

$R^5$ denotes $C_{1-3}$ alkyl,

$R^6$ means H or phenyl and

$R^7$ means H, methyl, tert.-butyl or trifluoromethyl.

**3.** Substituted 2,4-imidazolidinedione compounds of the formula I according to claim 1 or 2, characterised in that

$R^1$ means ethyl or cyclobutyl,

$R^2$ is phenyl or

$R^1$ and $R^2$ together mean $-(CH_2)_5-$.

**4.** Substituted 2,4-imidazolidinedione compounds of the formula I according to one of claims 1 to 3, characterised in that

$R^1$ and $R^2$ together mean $-(CH_2)_5-$.

**5.** Substituted 2,4-imidazolidinedione compounds of the formula I according to one of claims 1 to 4, characterised in that

$R^3$ means H, $C_{1-3}$ alkyl or $-CH_2-COOR^5$ and

$R^5$ is ethyl.

**6.** Substituted 2,4-imidazolidinedione compounds of the formula I according to claim 5, characterised in that

$R^3$ means H.

**7.** Substituted 2,4-imidazolidinedione compounds of the formula I according to one of claims 1 to 6, characterised in that

$R^4$ means pyridin-4-yl, pyridin-3-yl, thiazol-2-yl, 3-methylisoxazol-5-yl or 5-methylisoxazol-3-yl.

**8.** Substituted 2,4-imidazolidinedione compounds of the formula I according to claim 7, characterised in that

$R^4$ is thiazol-2-yl.

**9.** Process for the production of a substituted 2,4-imidazolidinedione compound of the formula I according to claim 1

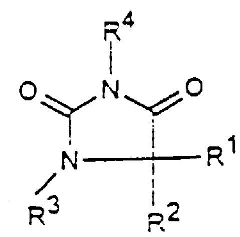

characterised in that 1,1'-carbonyldiimidazole or diphenyl carbonate are added to an amine of the formula II

$$R^4-NH_2$$

and then reacted with a compound of the formula III

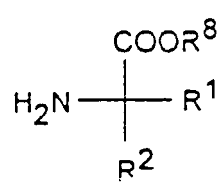

in which $R^8$ denotes H or $C_{1-3}$ alkyl
to yield a compound of the formula I in which $R^3$ means H,
which compound is then, if desired, deprotonated and then reacted with a compound of the formula IV

$$X\text{-}C_{1\text{-}5}\text{ alkyl}$$

or a compound of the formula V

$$X\text{- }(CH_2)_{1\text{-}4}\text{-}COOR^5$$

in which X means Cl, Br or I, to yield a compound of the formula I in which $R^3$ means $C_{1-5}$ alkyl or $-(CH_2)_{1-4}$-$COOR^5$.

**10.** Substituted 2,4-imidazolidinedione compounds of the formula I according to claim 1 for use as an active ingredient in a pharmaceutical preparation.

**11.** Substituted 2,4-imidazolidinedione compounds of the formula I according to claim 1 for use according to claim 10, characterised in that the pharmaceutical preparation is an immunomodulator.

**12.** Substituted 2,4-imidazolidinedione compounds of the formula I accdrding to claim 1 for use according to claim 10, characterised in that the pharmaceutical preparation has an antivasculitic action.

## Revendications

**1.** Imidazolidine-2,4-diones substituées de formule I

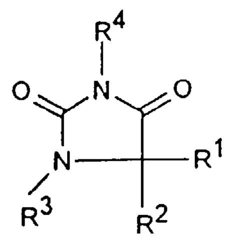

dans laquelle

$R^1$ est un groupe alkyle en $C_1$ à $C_6$ ou cycloalkyle en $C_3$ à $C_6$,
$R^2$ est un groupe alkyle en $C_1$ à $C_6$, phényle, $-(CH_2)_{1-3}$-phényle ou - $(CH_2)_{1-4}$-$COOR^5$, ou bien
$R^1$ et $R^2$ forment conjointement un groupe $-(CH_2)_{4-6}$-, $-(CH_2)_2$-O-$(CH_2)_2$- ou

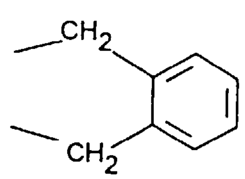

$R^3$ représente H, un groupe alkyle en $C_1$ à $C_5$ ou - $-(CH_2)_{1-4}$-$COOR^5$,
$R^4$ est un radical hétéroaromatique choisi dans le groupe des formules :

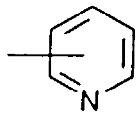 , 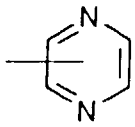 , 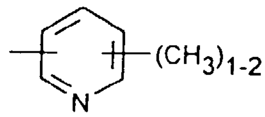 , 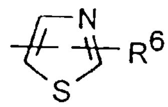 ,

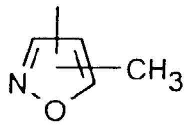 , 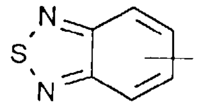 , 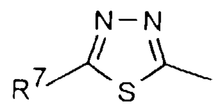

$R^5$ est un groupe alkyle en $C_1$ à $C_3$,
$R^6$ représente H, un groupe alkyle en $C_1$ à $C_4$, phényle ou benzyle et
$R^7$ représente H, un groupe alkyle en $C_1$ à $C_4$ ou trifluorométhyle.

**2.** Imidazolidine-2,4-diones substituées de formule I suivant la revendication 1, caractérisées en ce que $R^1$ est un groupe alkyle en $C_1$ à $C_4$ ou cycloalkyle en $C_3$ ou $C_4$, $R^2$ est un groupe alkyle en $C_3$ à $C_6$, phényle, $-(CH_2)_{1-2}$-phényle ou $-(CH_2)_{1-2}-COOR^5$, ou bien

$R^1$ et $R^2$ forment conjointement un groupe $-(CH_2)_5-$ ou

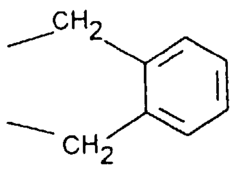

$R^3$ représente H, un groupe alkyle en $C_1$ à $C_4$ ou $-(CH_2)_{1-2}-COOR^5$,
$R^4$ est un radical hétéroaromatique du groupe des formules

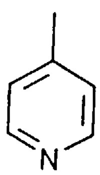 , 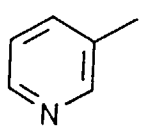 , 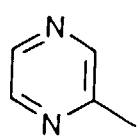 , 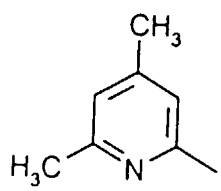 , 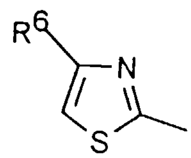 ,

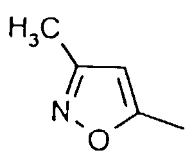 , 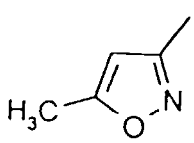 , 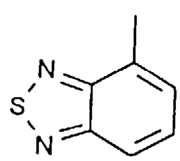 , 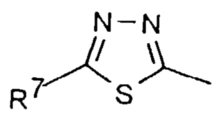

$R^5$ est un groupe alkyle en $C_1$ à $C_3$,
$R^6$ représente H ou un groupe phényle,
$R^7$ représente H, un groupe méthyle, tertiobutyle ou trifluorométhyle.

3. Imidazolidine-2,4-diones substituées de formule I suivant la revendication 1 ou 2, caractérisées en ce que

$R^1$ est un groupe éthyle ou cyclobutyle,
$R^2$ est un groupe phényle, ou bien
$R^1$ et $R^2$ forment conjointement un groupe $-(CH_2)_5-$.

4. Imidazolidine-2,4-diones substituées de formule I suivant l'une des revendications 1 à 3, caractérisées en ce que

$R^1$ et $R^2$ forment ensemble un groupe $-(CH_2)_5-$.

5. Imidazolidine-2,4-diones substituées de formule I suivant l'une des revendications 1 à 4, caractérisées en ce que

$R^3$ représente H, un groupe alkyle en $C_1$ à $C_3$ ou $-CH_2-COOR^5$ et
$R^5$ est un groupe éthyle.

6. Imidazolidine-2,4-diones substituées de formule I suivant la revendication 5, caractérisées en ce que $R^3$ représente H.

7. Imidazolidine-2,4-diones substituées de formule I suivant l'une des revendications 1 à 6, caractérisées en ce que

$R^4$ est un groupe pyridine-4-yle, pyridine-3-yle, thiazole-2-yle, 3-méthylisoxazole-5-yle ou 5-méthylisoxazole-3-yle.

8. Imidazolidine-2,4-diones substituées de formule I suivant la revendication 7, caractérisées en ce que $R^4$ est un groupe thiazole-2-yle.

9. Procédé de production d'une imidazolidine-2,4-dione substituée de formule I suivant la revendication 1,

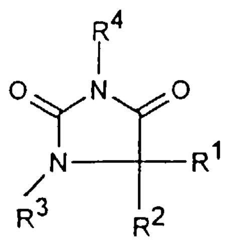

caractérisé en ce que
on ajoute à une amine de formule II

$$R^4\text{-}NH_2$$

du 1,1'-carbonyldiimidazole ou du carbonate de diphényle puis on fait réagir avec un composé de formule III

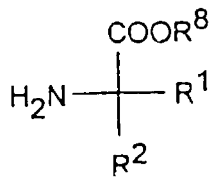

dans laquelle $R^8$ représente H ou un groupe alkyle en $C_1$ à $C_3$ pour obtenir un composé de formule I dans laquelle $R^3$ représente H,
que l'on déprotone le cas échéant et que l'on fait ensuite réagir avec un composé de formule IV

$$X\text{-}C_{1\text{-}5}\text{-alkyle}$$

ou avec un composé de formule V

$$X\text{- }(CH_2)_{1\text{-}4}\text{-}COOR^5$$

formules dans lesquelles X représente Cl, Br ou I, pour obtenir un composé de formule I dans laquelle $R^3$ est un groupe alkyle en $C_1$ à $C_5$ ou $-(CH_2)_{1-4}-COOR^5$.

**10.** Imidazolidine-2,4-diones substituées de formule I suivant la revendication 1, destinées à être utilisées comme substance active dans un médicament.

**11.** Imidazolidine-2,4-diones substituées de formule I suivant la revendication 1, destinées à être utilisées suivant la revendication 10, caractérisées en ce que le médicament est un immunomodulateur.

**12.** Imidazolidine-2,4-diones substituées de formule I suivant la revendication 1, destinées à être utilisées suivant la revendication 10, caractérisées en ce que le médicament a un effet anti-vascularites.